(19) Europäisches Patentamt
European Patent Office
Office européen des brevets

(11) Veröffentlichungsnummer: **0 193 066**
**B1**

# (12) EUROPÄISCHE PATENTSCHRIFT

(45) Veröffentlichungstag der Patentschrift: **04.01.89**

(51) Int. Cl.⁴: **C 07 D 279/02**, C 07 D 495/02

(21) Anmeldenummer: **86101989.1**

(22) Anmeldetag: **17.02.86**

(54) **Neue Ketosultame und Verfahren zu ihrer Herstellung.**

(30) Priorität: **23.02.85 DE 3506435**

(43) Veröffentlichungstag der Anmeldung: **03.09.86 Patentblatt 86/36**

(45) Bekanntmachung des Hinweises auf die Patenterteilung: **04.01.89 Patentblatt 89/1**

(84) Benannte Vertragsstaaten: **DE FR GB NL**

(56) Entgegenhaltungen:
**DE-A-2 556 620**

**LIEBIGS ANNALEN DER CHEMIE, Band 1985, Nr. 3, März 1985, Seiten 579-588, VCH Verlagsgesellschaft mbH, Weinheim, DE; A. BENDER et al.: "Reaktionen von 2-Oxosulfonamiden mit Carbonylverbindungen"**

(73) Patentinhaber: **HOECHST AKTIENGESELLSCHAFT, Postfach 80 03 20, D-6230 Frankfurt am Main 80 (DE)**

(72) Erfinder: **Bender, Albert, Dr. Dipl.- Chem., Henry-Moisand- Strasse 16, D-6500 Mainz- Laubenheim (DE)**
Erfinder: **Günther, Dieter, Dr. Dipl.- Chem., Nachtigallenweg 1 a, D-6233 Kelkheim (DE)**

**EP 0 193 066 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die vorliegende Erfindung bezieht sich auf neue Ketosultame und auf ein Verfahren zu ihrer Herstellung. Die erfindungsgemäßen Ketosultame besitzen die allgemeine Formel I

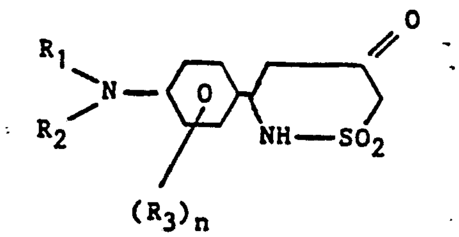

I

worin

$R_1$ und $R_2$ gleich oder verschieden sind und Wasserstoff, gegebenenfalls durch Halogen, Hydroxy-, Cyano- oder ($C_1$ bis $C_4$)-Al-koxygruppen ein- oder mehrfach substituiertes ($C_1$ bis $C_8$)-Alkyl oder Phenyl,
$R_3$ Wasserstoff oder eine ($C_1$ bis $C_4$)-Alkyl- oder -Alkoxygruppe,
n gleich 1 bis 4 oder
$R_1$, $R_2$ und $R_3$ zusammen mit dem zugehörigen Aminophenylrest Julolidinyl oder N-($C_1$ bis $C_3$-alkylcarbazol-3-yl
bedeuten.

Vorzugsweise werden Verbindungen beansprucht, in denen $R_1$ Methyl, Ethyl oder ω-Chlorethyl,
$R_2$ Methyl oder Ethyl,
$R_3$ Wasserstoff oder Methoxy
n gleich 1 oder
$R_1$, $R_2$ und $R_3$ zusammen mit dem zugehörigen Aminophenylrest Julolidinyl
bedeuten.

Gegenstand der Erfindung ist auch ein Verfahren zur Herstellung der Ketosultame der allgemeinen Formel I, das dadurch gekennzeichnet ist, daß man 4-Aminobenzaldehyde der Formel II

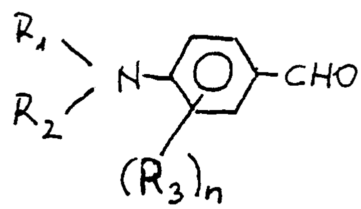

II

mit den für $R_1$, $R_2$, $R_3$ und n genannten Bedeutungen mit 2-Oxopropansulfonamid in einem sauren Lösungsmittel umsetzt, das Reaktionsprodukt isoliert und durch Umkristallisieren reinigt. Als saures Lösungsmittel dient vorzugsweise chlorwasserstoffgesättigter, niederer aliphatischer Alkohol. Weiterhin ist als säurehaltiges Lösungsmittel wäßrige Salzsäure mit einer Konzentration von mindestens 20 Gewichtsprozent geeignet.

Die Isolierung kann erfolgen durch Neutralisieren der sauren Lösung mit einer Base, wobei gegebenenfalls die Lösung vorher durch Abdestillieren des Lösungsmittels aufkonzentriert werden kann.

Als Basen kommen anorganische Basen, wie Alkali- oder Erdalkalicarbonate oder Alkali- oder Erdalkalihydrogencarbonate oder Alkali- oder Erdalkalihydroxide in Betracht.

Die erfindungsgemäßen Verbindungen der Formel I fallen dann gewöhnlich aus der Reaktionsmischung aus. Sie können durch Abfiltrieren isoliert und durch Umkristallisation gereinigt werden.

Aus entsprechend substituierten 4-Aminobenzaldehyden der Formel II und dem aus DE-OS 3 116 129 bekannten 2-Oxopropansulfonamid werden die erfindungsgemäßen Ketosultame durch Umsetzung der Reaktionspartner in mit Chlorwasserstoff gesättigtem Ethanol bei einer Temperatur, die zwischen 0° C und 80 ° C, vorzugsweise zwischen 20° C und 40° C liegt, hergestellt.

Die erfindungsgemäßen Ketosultame können als Zwischenprodukte zur Herstellung sulfonhaltiger Styrolderivate verwendet werden, die wiederum als Sensibilisatoren oder Farbstoffe dienen.

Die Erfindung wird durch die folgenden Beispiele näher erläutert.

**Beispiel 1 bis 4**

0,1 mol 2-Oxopropansulfonamid und 0,1 mol des entsprechenden 4-Aminobenzaldehyds (II) wurden vier Stunden in 100 ml mit Chlorwasserstoff gesättigem Ethanol (bei 30° C) gerührt. Dann wurde das Lösungsmittel

abdestilliert, der Rückstand in Wasser gelöst und mit Natriumbicarbonatlösung neutralisiert. Das Produkt wurde abfiltriert und aus Isopropanol umkristallisiert, wobei man farblose Kristalle erhielt. Die Charakterisierung der Produkte ist den nachfolgenden Tabellen zu entnehmen.

**Tabelle 1**

| Beispiel Nr. | $R_1$ | $R_2$ | $R_3$ | $F_p$ (°C) | Ausbeute % |
|---|---|---|---|---|---|
| 1 | ω-Chlorethyl | Methyl | H | 168-170 | 47 |
| 2 | Ethyl | Ethyl | H | 168 | 71 |
| 3 | Julolidinyl | | | 173-177 | 32 |
| 4 | Methyl | Methyl | 2-Methoxy | 205-208 | 54 |

Für die genannten Verbindungen ergab die Elementaranalyse folgende Werte:

**Tabelle 2**

| Beispiel | Summenformel (Molmasse) Ber.: Gef.: | C | H | N | S | Cl |
|---|---|---|---|---|---|---|
| 1 | $C_{14}H_{19}N_2SO_3Cl$ | 49,5 | 5,7 | 8,4 | 9,7 | 10,7 |
| | (331,66) | 49,4 | 5,7 | 8,3 | 10,0 | 10,6 |
| 2 | $C_{14}H_{20}N_2SO_3$ | 56,8 | 6,8 | 9,5 | 10,8 | - |
| | (296,21) | 56,9 | 6,9 | 9,7 | 10,7 | - |
| 3 | $C_{16}H_{20}N_2SO_3$ | 57,3 | 6,7 | 9,3 | 10,7 | - |
| | (300,32) | 57,6 | 6,7 | 9,1 | 10,6 | - |
| 4 | $C_{13}H_{18}N_2SO_3$ | 55,3 | 6,4 | 9,9 | 11,4 | - |
| | (282,20) | 55,2 | 6,5 | 9,9 | 11,2 | - |

Die Verbindungen lassen sich auch in wäßrigem Chlorwasserstoff herstellen, wie folgendes Beispiel zeigt:

**Beispiel 5**

0, 1 mol 2-Oxopropansulfonamid und 0,1 mol 4-Diethylaminobenzaldehyd wurden fünf Stunden in 80 ml 35 %-iger Salzsäure bei 30°C gerührt. Dann wurde mit Natronlauge bis zum pH = 7 neutralisiert. Der Niederschlag wurde abfiltriert, mit Wasser gewaschen und aus Isopropanol umkristallisiert. Es wurden 14 g (48 % Ausbeute) der in Tabelle 1 als Beispiel 2 aufgeführten Verbindung erhalten.

**Patentansprüche**

1. Ketosultame der allgemeinen Formel I

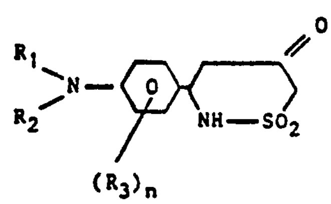

,

worin

$R_1$ und $R_2$ gleich oder verschieden sind und Wasserstoff, gegebenenfalls durch Halogen, Hydroxy-, Cyano- oder ($C_1$ bis $C_4$)-Alkoxygruppen ein- oder mehrfach substituiertes ($C_1$ bis $C_8$)-Alkyl oder Phenyl,

$R_3$ Wasserstoff oder eine ($C_1$ bis $C_4$) Alkyl- oder Alkoxygruppe,

n gleich 1 bis 4 oder

$R_1$, $R_2$ und $R_3$ zusammen mit dem zugehörigen Aminophenylrest Julolidinyl oder N-($C_1$ bis $C_3$)-alkylcarbazol-3-yl

bedeuten.

2. Ketosultame nach Anspruch 1, worin
$R_1$ Methyl, Ethyl oder ω-Chlorethyl,
$R_2$ Methyl oder Ethyl,
$R_3$ Wasserstoff oder Methoxy)
n gleich 1 oder
$R_1$ $R_2$ und $R_3$ zusammen mit dem zugehörigen Aminophenylrest Julolidinyl
bedeuten.

3. Verfahren zur Herstellung der Ketosultame der allgemeinen Formel I nach Anspruch 1, dadurch gekennzeichnet, daß man 4-Aminobenzaldehyde der Formel II

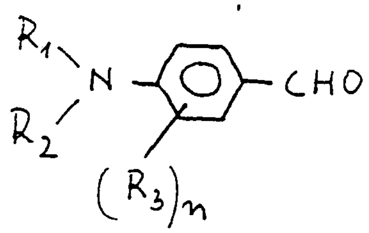

II

mit den in Anspruch 1 für $R_1$, $R_2$, $R_3$ und n genannten Bedeutungen mit 2-Oxopropansulfonamid in einem sauren Lösungsmittel umsetzt, das Reaktionsprodukt isoliert und durch Umkristallisieren reinigt.

4. Verfahren nach Anspruch 3, dadurch gekennzeichnet, daß man als saures Lösungsmittel einen mit Chlorwasserstoff gesättigten niederen aliphatischen Alkohol verwendet.

5. Verfahren nach Anspruch 4, dadurch gekennzeichnet, daß man als niederen Alkohol Ethanol verwendet.

**Claims**

1. Ketosultams of the general formula I

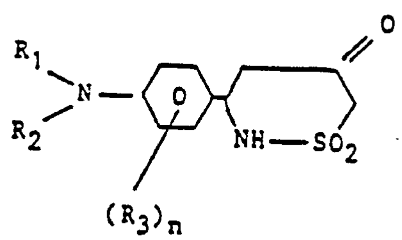

I

in which

$R_1$ und $R_2$ are identical or different and denote hydrogen, ($C_1$ to $C_8$)-alkyl, which is optionally substituted by one or several halogen atoms, hydroxy groups, cyano groups or ($C_1$ to $C_4$)-alykox groups, or phenyl,
$R_3$ denotes hydrogen or a ($C_1$ bis $C_4$)-alkyl group or ($C_1$-$C_4$)-alkyl group,
n is a number from 1 to 4, or
$R_1$, $R_2$ and $R_3$, together with the corresponding amino-phneyl group, denote julolidinyl or N-($C_1$ to $C_3$)-alkylcarbazol-3-yl.

2. Ketosultams as claimed in Claim 1, wherein
$R_1$ denotes methyl, ethyl oder -chloroethyl,
$R_2$ denotes methyl or ethyl,
$R_3$ denotes hydrogen or methoxy,
n is 1, or
$R_1$, $R_2$ and $R_3$, together with the corresponding amino-phenyl group, denote julolidinyl.

3. Process for the preparation of the ketosultams of the general formula I, according to Claim 1, wherein 4-aminobenzaldehydes of the formula II

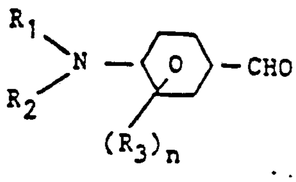

II

in which $R_1$, $R_2$, $R_3$ and n have the meanings indicated in Claim 1, are reacted, in an acid solvent, with 2-oxopropane sulfonamide and the reaction product is isolated and purified by recrystallization.

4. Process as claimed in Claim 3, wherein the acid solvent is a lower aliphatic alcohol saturated with hydrogen chloride.

5. Process as claimed in Claim 4, wherein the lower alcohol is ethanol.

**Revendications**

1. Cétosultames de formule générale I

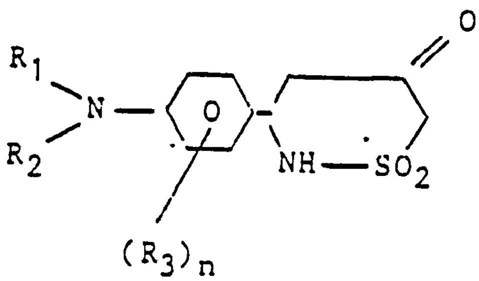

I

dans laquelle

$R_1$ et $R_2$ sont identiques ou différents, et représentent un atome d'hydrogène ou un radical alkyle en $C_1$-$C_8$ ou phényle, éventuellement substitué une ou plusieurs fois par des atomes d'halogène ou par des groupes hydroxy, cyano ou alcoxy en $C_1$-$C_4$;

$R_3$ représente un atome d'hydrogène ou un groupe alkyle ou alcoxy en $C_1$-$C_4$;

n vaut de 1 à 4; ou

$R_1$, $R_2$ et $R_3$ forment ensemble, avec le reste aminophényle correspondant) un radical julolidinyle ou N-alkyl-($C_1$-$C_3$)-carbazol-3-yle.

2. Cétosultames selon la revendication 1, dans lesquels

$R_1$ représente le groupe méthyle, éthyle ou ω-chloréthyle;

$R_2$ représente le groupe méthyle ou éthyle;

$R_3$ représente un atome d'hydrogène ou le groupe méthoxy; n est égal à 1; ou

$R_1$, $R_2$ et $R_3$ forment ensemble, conjointement avec le reste aminophényle, un radical julolidinyle.

3. Procédé pour la préparation des cétosultames de formule générale 5 selon la revendication 1, caractérisé en ce que l'on fait réagir avec le 2-oxopropanesulfonamide, dans un solvant acide, des 4-aminobenzaldéhydes de formule II

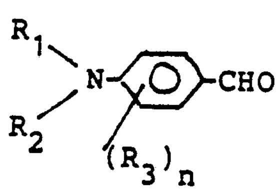

II

dans laquelle $R_1$, R2, $R_3$ et n ont les significations données plus haut, on isole le produit de réaction et on le purifie par recristallisation.

4. Procédé selon la revendication 3, caractérisé en ce que l'on utilise en tant que solvant acide un alcool aliphatique inférieur saturé avec de l'acide chlorhydrique.

5. Procédé selon la revendication 4, caractérisé en ce que l'on utilise en tant qu'alcool inférieur l'éthanol.

FORMELTABELLE

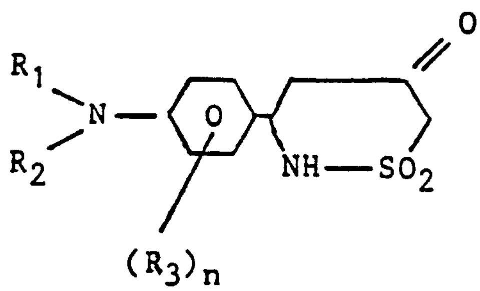

I

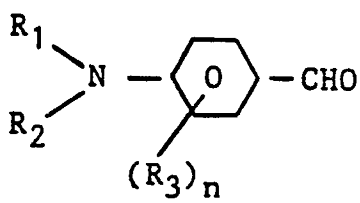

II

$$CH_3 - \overset{O}{\overset{\|}{C}} - CH_2 - SO_2 - NH_2$$

III